# EUROPEAN PATENT APPLICATION

(11) **EP 3 960 228 A1**
(43) Date of publication of application: **02.03.2022**
(21) Application number: 20795886.9
(22) Date of filing: 24.04.2020
(51) Int. Cl.: A61M 35/00, A61M 37/00

(54) **APPLICATOR 2 OF WATER-SOLUBLE SHEET-LIKE FORMULATION**

(30) Priority: 25.04.2019 JP 2019083868
(71) Applicant: Cosmed Pharmaceutical Co., Ltd., Kyoto-shi, Kyoto 601-8014 (JP)
(72) Inventor: QUAN, Ying-shu, Kyoto-shi, Kyoto 601-8014 (JP); TANAKA, Hiroshi, Kyoto-shi, Kyoto 601-8014 (JP); KAMIYAMA, Fumio, Kyoto-shi, Kyoto 601-8014 (JP)
(74) Representative: Ter Meer Steinmeister & Partner
(86) International application number: PCT/JP2020/017649
(87) International publication number: WO 2020/218486

(57) **Abstract**

Provided is a further stable method of moisture supply from an applicator having a nozzle attached thereto and configured to supply moisture from the back surface of a water-soluble sheet-like formulation. A nozzle for hold and dissolution of a water-soluble sheet-like formulation, the nozzle including a nozzle having an end portion having a flat surface having a size such that a water-soluble sheet-like formulation can be held, the end portion having at least one hole, the flat surface being smooth excluding the at least one hole, and ring-like foam disposed around a periphery of the end portion of the nozzle; a nozzle for hold and dissolution of a water-soluble sheet-like formulation, the nozzle including a nozzle having an end portion having a flat surface having a size such that a water-soluble sheet-like formulation can be held, the end portion having at least one hole, the flat surface having an uneven portion excluding the at least one hole, and ring-like foam disposed around a periphery of the end portion of the nozzle; and an applicator of a water-soluble sheet-like formulation, the applicator including a liquid container and the nozzle for hold and dissolution of a water-soluble sheet-like formulation.

## Description

### TECHNICAL FIELD

The present invention relates to a nozzle for an applicator to be used for application of a water-soluble sheet-like formulation to skin, and relates to an applicator equipped with the nozzle.

### BACKGROUND ART

As a method of administering a drug to a human body, oral administration and transdermal administration are often used. Injection is a representative transdermal administration method, but is bothersome and painful. In transdermal administration, the stratum corneum serves as a barrier against drug permeation, so that the permeability is not necessarily sufficient if the drug is only simply applied to the skin surface. However, the drug permeation efficiency can be remarkably improved, as compared with the application method, by perforating the stratum corneum using a minute needle, that is, a microneedle. A large number of such microneedles are collected on a substrate to obtain a microneedle array. Furthermore, a product easy to use called a microneedle patch is obtained by adding a pressure sensitive adhesive sheet for adhesion of the microneedle array to the skin, a protective release sheet for protection of the pressure sensitive adhesive sheet and for support in attachment of the microneedle array to the skin, and the like.

When administered to the skin, the microneedle array is not easily inserted into the skin only by pressing the microneedle array with a finger. This is because the skin is generally a flexible and elastic tissue, and therefore even if pressed with the sharp tip of the microneedle, the skin absorbs the impact and deforms to prevent the microneedle from entering the skin.

For administration of the microneedle array to the skin having impact absorbing ability, it is appropriate to impact the microneedle array in the direction toward the skin. As a specific method of impacting the microneedle array, methods have been proposed in which a spring (in Patent Documents 1 to 6, and 8), air pressure (in Patent Document 5), magnetic force (in Patent Document 7), or the like is used in a microneedle administration device (microneedle applicator). In order to allow women and children to use the spring easily, the method of compressing the spring and the method of triggering are to be devised. The air pressure and the magnetic force also cannot necessarily be used simply. There remain problems in practical use of conventional microneedle array administration devices, and users have awaited a device that is further simple and can realize insertion reliably.

A microneedle prepared with a water-soluble material dissolves in moisture in the skin after insertion, but the dissolution takes a considerable time to impair convenience for the subject significantly particularly in the case of a cosmetic product as an object. If, after insertion of a microneedle array prepared with a water-soluble material into the skin, moisture is supplied from the back surface of the microneedle array, the microneedle rapidly dissolves (see Patent Document 9). The present inventors have developed a cosmetic hyaluronic acid gel sheet using hyaluronic acid, which is a water-soluble polymer, as a raw material, and have reported that penetration of hyaluronic acid and a blending component into the skin is promoted by massage in which the gel sheet is applied to the skin, then a small amount of water is added to the gel sheet, and the skin is massaged (see Patent Document 10).

The present inventors have developed an applicator capable of reliably administering a water-soluble sheet-like formulation to the skin at various sites and supplying moisture from the back surface of the water-soluble sheet-like formulation (see Patent Document 11).

### PRIOR ART DOCUMENT

### PATENT DOCUMENT

Patent Document 1: JP 2004-510530 T (Japanese Patent No. 4198985)
Patent Document 2: JP 2004-510534 T (Japanese Patent No. 4104975)
Patent Document 3: JP 2004-510535 T (Japanese Patent No. 4659332)
Patent Document 4: JP 2005-533625 T
Patent Document 5: JP 2006-500973 T
Patent Document 6: JP 2007-509706 T (Japanese Patent No. 4682144)
Patent Document 7: JP 2011-078711 A
Patent Document 8: JP 2014-42788 A
Patent Document 9: JP 2011-194189 A (Japanese Patent No. 5408592)
Patent Document 10: JP 2014-024828 A (Japanese Patent No. 5840107)
Patent Document 11: JP 2018-164688 A

### SUMMARY OF THE INVENTION

### PROBLEM TO BE SOLVED BY THE INVENTION

Microneedle arrays and cosmetic gel sheets including a water-soluble polymer as a raw material are a flexible sheet-like formulation having a thickness of about several hundred µm for improvement in adhesion to the skin. When applied to the skin, such a water-soluble sheet-like formulation may absorb moisture of the fingertip and adhere to the finger more than to the target skin, or when the water-soluble sheet-like formulation is applied to the scalp, the hair may hinder the adhesion, and the formulation may be detached before being absorbed by the scalp. The present inventors have developed a nozzle that facilitates adhesion and release of a water-soluble sheet-like formulation. Furthermore, attachment of the nozzle to various liquid containers led to success in development of an applicator with which application of a water-soluble sheet-like formulation to the skin and dissolving operation can be instantaneously performed (see Patent Document 11).

However, in supply of moisture from the back surface of a water-soluble sheet-like formulation with the applicator to which the nozzle is attached, appropriate moisture supply is sometimes prevented without appropriate adjustment of the degree of adhesion of the nozzle to the skin. More specifically, there is a possibility that moisture is not sufficiently supplied to the skin with the nozzle pressed against the skin too much, and water flows down along the skin due to an inappropriate angle of the nozzle to the skin.

An object of the present invention is to provide a further stable method of moisture supply from an applicator having a nozzle attached thereto and configured to supply moisture from the back surface of a water-soluble sheet-like formulation.

Here, examples of the water-soluble sheet-like formulation in the present invention include not only microneedle arrays but also sheet-like formulations intended to be rapidly dissolved in moisture after application to the skin and absorbed into the skin, such as cosmetic water-soluble gel sheets. The water-soluble sheet-like formulation is prepared using a water-soluble polymer as a base, and has a thickness of about 1 mm or less. Examples of the water-soluble polymer include hyaluronic acid (and sodium salts thereof) and derivatives thereof, collagen, hydroxypropyl cellulose, chondroitin sulfate, dextran, proteoglycan, and mixtures of these water-soluble polymers. The term "back surface" of a water-soluble sheet-like formulation refers to the surface opposite from the surface to be adhered to the skin.

### MEANS FOR SOLVING THE PROBLEM

In order to solve the above-described problem, the present inventors intensively studied, and as a result, have succeeded in developing a nozzle that facilitates adhesion and release of a water-soluble sheet-like formulation. The present inventors have found that supply of moisture to the skin is further stabilized with the nozzle in which the circumference of the nozzle end as an outlet of a liquid is surrounded by foam, and attached the nozzle to various liquid containers. Thus, a device has been developed with which application of a water-soluble sheet-like formulation to the skin and dissolving operation can be appropriately performed without an error, leading to completion of the present invention.

The present invention is as follows.
[1] A nozzle for hold and dissolution of a water-soluble sheet-like formulation, the nozzle including: a nozzle having an end portion having a flat surface having a size such that a water-soluble sheet-like formulation can be held, the end portion having at least one hole, the flat surface being smooth excluding the at least one hole; and ring-like foam disposed around a periphery of the end portion of the nozzle.
[2] A nozzle for hold and dissolution of a water-soluble sheet-like formulation, the nozzle including: a nozzle having an end portion having a flat surface having a size such that a water-soluble sheet-like formulation can be held, the end portion having at least one hole, the flat surface having an uneven portion excluding the at least one hole; and ring-like foam disposed around a periphery of the end portion of the nozzle.
[3] The nozzle for hold and dissolution of a water-soluble sheet-like formulation according to the item [2], wherein a level difference in the uneven portion is 0.1 to 3.0 mm.
[4] The nozzle for hold and dissolution of a water-soluble sheet-like formulation according to the item [2] or [3], wherein the uneven portion forms a groove extending from the at least one hole toward the periphery of the end portion.
[5] The nozzle for hold and dissolution of a water-soluble sheet-like formulation according to any one of the items [2] to [4], wherein the periphery of the end portion is surrounded by a protrusion higher than the uneven portion.
[6] The nozzle for hold and dissolution of a water-soluble sheet-like formulation according to any one of the items [1] to [5], wherein the ring-like foam has an upper end higher than the periphery of the end portion of the nozzle by 0.1 to 10 mm in a state that the end portion of the nozzle is directed upward and the periphery of the end portion is in a horizontal direction.
[7] The nozzle for hold and dissolution of a water-soluble sheet-like formulation according to any one of the items [1] to [6], wherein the nozzle includes a synthetic resin selected from the group consisting of polyethylene resins, polypropylene resins, soft polyolefin resins, polyurethane resins, polyvinyl chloride, soft polyvinyl chloride resins, and silicone resins.
[8] The nozzle for hold and dissolution of a water-soluble sheet-like formulation according to any one of the items [1] to [7], wherein the nozzle includes a synthetic resin having self-viscosity.
[9] The nozzle for hold and dissolution of a water-soluble sheet-like formulation according to any one of the items [1] to [8], wherein the nozzle includes a synthetic resin having self-viscosity, the synthetic resin selected from the group consisting of soft polyolefin resins, polyurethane resins, soft polyvinyl chloride resins, and silicone resins.
[10] The nozzle for hold and dissolution of a water-soluble sheet-like formulation according to any one of the items [1] to [9], wherein the ring-like foam includes a material selected from the group consisting of polyurethanes, polyolefins, phenolic resins, polyvinyl chloride, and urea resins or includes a mixture of materials selected from the group consisting of polyurethanes, polyolefins, phenolic resins, polyvinyl chloride, and urea resins.
[11] An applicator of a water-soluble sheet-like formulation, the applicator including a liquid container and the nozzle for hold and dissolution of a water-soluble sheet-like formulation according to any one of the items [1] to [10] .
[12] The applicator of a water-soluble sheet-like formulation according to the item [11], wherein the liquid container has an opening to which the nozzle is attached.
[13] The applicator of a water-soluble sheet-like formulation according to the item [11] or [12], configured to apply a water-soluble sheet-like formulation held in the end portion of the nozzle to the skin, and supply a liquid to a back surface of the water-soluble sheet-like formulation by pressing the liquid container.
[14] The applicator of a water-soluble sheet-like formulation according to the item [11], wherein the liquid container is equipped with a spraying device, and the spraying device has an end opening to which the nozzle is attached.
[15] The applicator of a water-soluble sheet-like formulation according to the claim [14], configured to apply a water-soluble sheet-like formulation held in the end portion of the nozzle to the skin, and supply a liquid to a back surface of the water-soluble sheet-like formulation by spraying the liquid from the spraying device.
[16] The applicator of a water-soluble sheet-like formulation according to the item [11], wherein the liquid container is equipped with a pump, and the pump has an end opening to which the nozzle is attached.
[17] The applicator of a water-soluble sheet-like formulation according to the item [16], configured to apply a water-soluble sheet-like formulation held in the end portion of the nozzle to the skin, and discharge a liquid to a back surface of the water-soluble sheet-like formulation by pushing the pump.

### EFFECT OF THE INVENTION

The nozzle for hold and dissolution of a water-soluble sheet-like formulation of the present invention (hereinafter, abbreviated to "nozzle with a foam ring edge of the present invention") enables rapid dissolution of a water-soluble sheet-like formulation by supplying water from the hole of the nozzle in a state that the end of the nozzle is pressed against the water-soluble sheet-like formulation attached to the skin. The present invention is characterized by a ring-like edge including foam around the nozzle. The ring edge includes open-cell foam, and allows air between the nozzle and the skin to be smoothly exhausted to the outside during supply of water from the nozzle hole. Therefore, delivery of supply of water to the skin behind a microneedle patch is much easier and smoother with the nozzle with a foam ring edge of the present invention than with a nozzle without a ring edge. The nozzle preferably has an end portion having an uneven portion, but the nozzle having a ring edge according to the present invention is not necessarily to have an uneven portion.

The applicator of the present invention includes the nozzle with a foam ring edge of the present invention and includes a liquid container, and any liquid container may be used as long as the opening of the liquid container and the nozzle can be fitted. When the applicator of the present invention is used for administration of a water-soluble microneedle array, the end of the nozzle is pressed against the microneedle array for an appropriate time, and thus the microneedle is reliably inserted into the skin. Furthermore, in a state that the end of the nozzle is pressed against the microneedle array, water is supplied from the liquid container to the entire back surface of the array uniformly, and thus the microneedle array rapidly dissolves. At this time, water is accurately and uniformly supplied to the back of the microneedle array with the aid of the foam ring edge, and inconvenience, for example, such that water flows down along the skin is eliminated. When the applicator of the present invention is used for application of a cosmetic water-soluble gel sheet, water in the liquid container is supplied from the back surface in a state that the end of the nozzle is pressed against the gel sheet, and thus the gel sheet rapidly dissolves and spreads on the skin surface to promote moisturization of the skin and absorption of the valuable ingredient.

### BRIEF DESCRIPTION OF THE DRAWINGS

FIG. 1 shows a sectional view and a bottom view of a nozzle in Example 1.
FIG. 2 is a view of an applicator in which the nozzle in Example 1 is attached to a polyethylene liquid container.
FIG. 3 shows examples of a nozzle (A, B, C). The left views are a bottom view, and the right views are a sectional view. For simplification, ring-like foam is omitted.
FIG. 4 shows examples of an applicator in which a nozzle is attached to a liquid container (A, B, C).

### MODE FOR CARRYING OUT THE INVENTION

The nozzle with a foam ring edge of the present invention includes: a nozzle having an end portion having a flat surface having a size such that a water-soluble sheet-like formulation can be held, the end portion having at least one hole, the flat surface being smooth or having an uneven portion excluding the at least one hole (hereinafter, sometimes abbreviated to "nozzle in the present invention"); and ring-like foam disposed around a periphery of the end portion of the nozzle (hereinafter, sometimes abbreviated to "foam in the present invention").

The nozzle in the present invention is a moisture supplying member having an object of dissolution of a water-soluble sheet-like formulation. The nozzle has an end portion having a flat surface having a size such that a water-soluble sheet-like formulation can be held. In the nozzle in the present invention, the flat surface of the end portion can have a shape depending on the size of a water-soluble sheet-like formulation, and the shape may be a circle, an ellipse, a comma shape, or a polygon such as a triangle, a quadrangle, a pentagon, or a hexagon. The main body of the nozzle of the present invention may have a cylindrical shape, an elliptical cylindrical shape, or a polygonal column shape depending on the shape of the flat surface of the end portion. The main body preferably has a cylindrical shape to be fitted into an opening of various liquid containers.

The periphery of the end portion of the nozzle in the present invention is preferably surrounded by a protrusion higher than the smooth flat surface or the uneven portion so that the supplied water does not overflow. The protrusion surrounding the periphery serves as a weir that prevents the water spreading on the smooth flat surface or the water spreading through the groove formed by the uneven portion from escaping to the outside of the water-soluble sheet-like formulation.

The end portion of the nozzle in the present invention has at least one hole. The number of the at least one hole may be two or more for the purpose of introducing water from the liquid container to the back surface of the water-soluble sheet-like formulation, and the size of the at least one hole is not particularly limited. The at least one hole may be located at the center or in the peripheral portion of the flat surface of the end portion. The disposition and the interval of the plurality of holes are not particularly limited.

The flat surface of the end portion of the nozzle in the present invention may be smooth. However, for uniform supply of water to the end portion of the nozzle, an uneven portion is preferably formed on the flat surface excluding the at least one hole. The uneven portion may have any shape such as a spiral shape, a dot shape, or a radial shape as long as a water-soluble sheet-like formulation can be attached to and detached from the end portion of the nozzle and a groove can be formed through which water can be supplied from the hole to the entire end portion. Typically, the degree of unevenness is preferably such that the level difference in the uneven portion is 0.1 to 3.0 mm, and more preferably such that the level difference is 0.1 to 2.0 mm. If the level difference is 0.1 mm or less, supply of water to the surface through the hole is insufficiently rapid. If the level difference is 3.0 mm or more, water supplied through the hole is to have a large amount for spreading over the entire surface.

The area ratio of the protrusion or the flat surface portion to the groove portion or the recess is preferably 20 : 1 or more. If the area ratio of the recess is too small, the groove portion is so small that it is difficult to supply water to the entire bottom surface. If the area of the groove portion is too large, the flat surface of the flat surface portion of the nozzle is so small that a water-soluble sheet pressed against the skin with the nozzle may be insufficiently adhered to the nozzle because the degree of adhesion of the water-soluble sheet to the flat surface portion is insufficient. Therefore, the area ratio of the protrusion or the flat surface portion to the groove portion or the recess is preferably 1 : 10 or less.

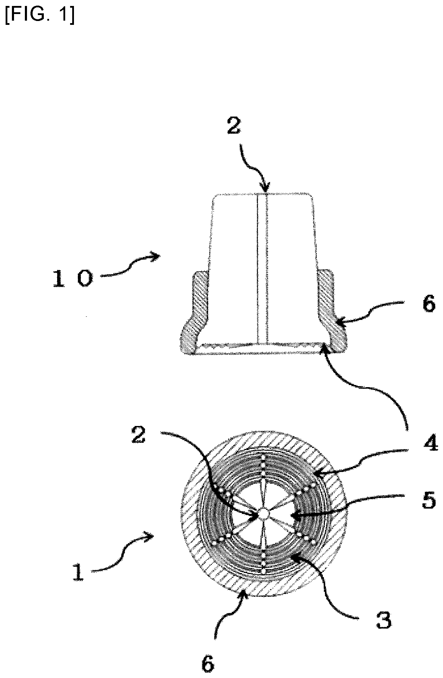

In the nozzle in the present invention, the uneven portion preferably forms a groove extending from the at least one hole toward the periphery of the end portion for smooth supply of an appropriate amount of water to the entire water-soluble sheet-like formulation. Here, the term "groove" refers to a low part formed by two adjacent protrusions and one recess surrounded by the two protrusions. The shape of the groove is not particularly limited. Specific examples of the groove include grooves in the end portion of the nozzle shown in FIG. 1, such as elongated grooves tracing concentric circles in an uneven portion 3 spread concentrically around a hole 2 in the central portion, and radial (fan-shaped) grooves 5 directly connected to the hole 2. In the nozzle, a protrusion 4 surrounding the periphery is also formed.

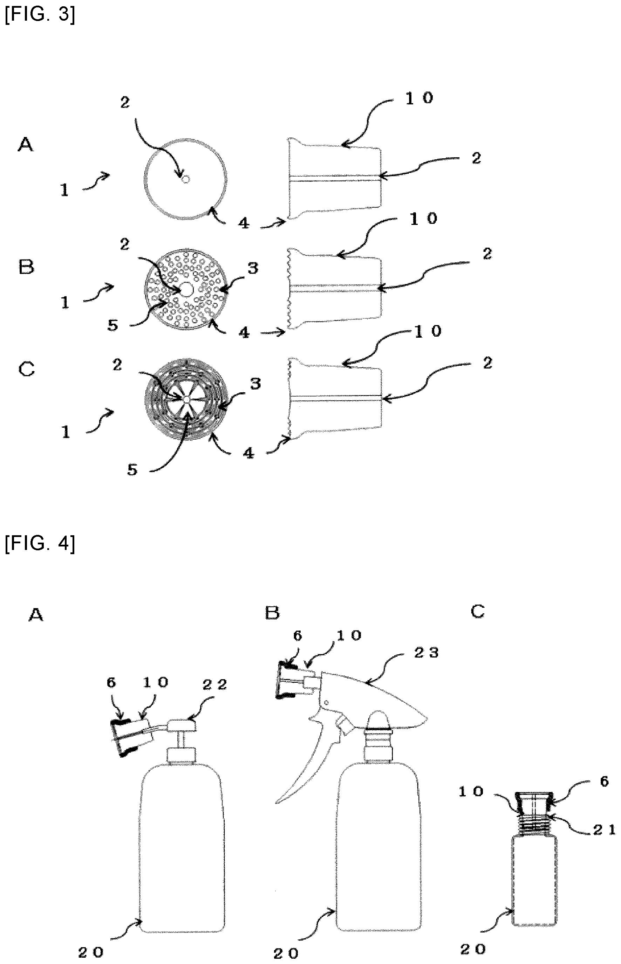

Another aspect of the nozzle in the present invention is shown in FIG. 3. In FIG. 3A, the flat surface of the end portion of the nozzle is smooth, and neither an uneven portion nor a groove is formed. In FIG. 3C, radial grooves 5 and grooves tracing concentric circles are formed. In FIG. 3B, a large number of dots are formed in an uneven portion 3, and dot-shaped grooves 5 are formed from a hole 2 toward the periphery.

The material of the nozzle in the present invention is not particularly limited, and various materials such as metals, ceramics, and plastic can be used. In consideration of ease of production, convenience for use, and the like, plastic is preferable, and among plastic, synthetic resins are more preferable such as polyolefin resins such as polyethylene resins and polypropylene resins, polyvinyl chloride, polyurethane resins, and silicone resins. In consideration of fitting with liquid containers of various materials and favorable contact feeling of the nozzle for the skin, soft polymer substances are preferable. The Young's modulus, which is a measure of the hardness of the nozzle material, is preferably 500 to 5 MPa. Such soft polymers have a glass transition temperature of room temperature or less, are in a rubber state at room temperature, and have self-adhesiveness and adhesiveness to the back surface of a microneedle patch. Here, the term "room temperature" refers to a temperature of 20 °C to 25 °C. The above-described property is referred to as self-viscosity. The material of the nozzle may be a synthetic resin having self-viscosity in which the polymer itself is hard as long as the resin composition including the synthetic resin and a plasticizer is soft. As a specific material, soft polyolefin resins, polyurethane resins, soft vinyl chloride resins, and silicone resins are preferable, and silicone resins are more preferable. The convenience from the fact that the nozzle has self-viscosity is the convenience such that the adhesive property to a water-soluble sheet-like formulation is improved, so that it is possible to apply a sheet-like formulation to the skin by adhering the sheet-like formulation to the nozzle with weak contact and pressing the sheet-like formulation against the skin in that state.

The nozzle including a synthetic resin can be produced with a known method such as a method in which a raw material resin is put into a mold and molded.

In the nozzle with a foam ring edge of the present invention, an edge including ring-like foam is disposed around the periphery of the end portion of the nozzle in the present invention. The ring-like foam is disposed so as to be higher than the smooth flat surface or the uneven portion, and than the protrusion surrounding the periphery so that the supplied water uniformly flows to the end portion of the nozzle and does not overflow. The ring-like foam around the periphery serves as a weir that prevents water spreading on the end portion from escaping to the outside of the water-soluble sheet-like formulation.

As the foam in the present invention, any resin foam can be molded and used. The resin foam to be used is open-cell foam. Open-cell foam is preferable because it has open cells, which are connected and communicating with each other. If the cell diameter is too small, air permeates the cells with difficulty, and if the cell diameter is too large, water also permeates the cells easily, and therefore the cell diameter is preferably 0.1 mm or more and 5 mm or less.

The foam in the present invention is disposed around the periphery of the end portion of the nozzle in the present invention. The foam has a shape of a ring having a space, at the center, fitted with the periphery of the end portion of the nozzle. The circumference of the ring-like shape is typically circular, but may be elliptical or polygonal. The shape of the space at the center can be appropriately set according to the shape of the flat surface of the end portion of the nozzle, and the shape may be a circle, an ellipse, a comma shape, or a polygon such as a triangle, a quadrangle, a pentagon, a hexagon, or an octagon.

The ring-like foam and the nozzle are to be in a relationship in which the upper end of the foam is higher than the periphery of the end portion of the nozzle. In a state that the end portion of the nozzle is directed upward and the periphery of the end portion is in a horizontal direction, the upper end of the foam is preferably higher than the periphery of the end portion of the nozzle by 0.1 mm to 10 mm, and more preferably by 0.3 mm to 5 mm.

The raw material of the foam in the present invention is not particularly limited, and examples of the raw material include synthetic resins. Examples of the synthetic resins include polyurethanes, polyolefins (such as polyethylene and polypropylene), phenol resins, polyvinyl chloride, urea resins, and mixtures of the above-described synthetic resins.

The size of the foam in the present invention can be set to any size depending on the shape of the nozzle to be disposed. In the case of a concentric doughnut shape having a circular space at the center, the difference between the radii of the two circles, that is, the size of the part around the nozzle is usually about 5 mm to 30 mm, and preferably 7 mm to 20 mm. Foam having another shape can be set in the same manner as the foam having a concentric doughnut shape.

Examples of the method of producing the foam in the present invention include a method in which a foam block having open cells is produced with a known chemical foaming method or physical foaming method, and the produced foam block is subjected to slicing, slitting, punching and cutting, or the like and molded.

The end portion of the nozzle in the present invention is put in the central space portion of the ring-like foam produced as described above, and the ring-like foam is fixed at a predetermined position. Thus, the nozzle with foam of the present invention can be obtained. In a state that the end portion of the nozzle is directed upward and the periphery of the end portion is in a horizontal direction, the ring-like foam is preferably fixed at a position such that the upper end of the ring-like foam is higher than the periphery of the end portion of the nozzle by 0.1 mm to 10 mm, and more preferably by 0.3 mm to 5 mm.

Examples of the method of fixing include fixing with an adhesive or the like, and fixing with a stopper provided more rearward than the end portion of the nozzle by the thickness of the ring-like foam.

In replacement of the ring-like foam fixed with an adhesive or the like, the ring-like foam is replaced together with the nozzle, and in replacement of the ring-like foam fixed with a stopper, only the ring-like foam can be replaced. In the latter case, the ring-like foam can be removed from the nozzle after each use and cleaned and dried to maintain the cleanliness of the foam. When only the ring-like foam deteriorates, the foam can be replaced in partial replacement.

The applicator of the present invention includes a liquid container and the nozzle with a foam ring edge of the present invention. In the combination of the liquid container and the nozzle with a foam ring edge, they may be separate from each other. In this case, the nozzle with a foam ring edge is attached to the opening of the liquid container at the time of use. The liquid container may be filled with a liquid in advance, or a liquid may be put into the liquid container immediately before use. In the present invention, the liquid is typically water, but may be a mixed liquid with a solvent other than water as long as the mixed liquid can dissolve the water-soluble sheet-like formulation. The liquid may be an aqueous solution additionally containing a medicinal ingredient or a valuable ingredient to be applied to the skin as necessary.

The material of the liquid container is not particularly limited, and various materials such as metals, ceramics, and plastic can be used in consideration of fitting with the nozzle. In consideration of ease of production, convenience for use, and the like, plastic is preferable, and among plastic, synthetic resins are more preferable such as polyolefin resins such as polyethylene resins and polypropylene resins, polyvinyl chloride, polyurethane resins, and silicone resins.

The applicator of the present invention has the nozzle with a foam ring edge of the present invention. Examples of the applicator include the applicator shown in FIG. 4C. In this case, the applicator can apply a water-soluble sheet-like formulation held in the end portion of the nozzle to the skin, and supply a liquid to the back surface of the water-soluble sheet-like formulation by pressing the liquid container. The amount of the liquid to be supplied can be appropriately set according to, for example, the size (area and thickness) of the water-soluble sheet-like formulation. The amount of the liquid can be set by adjusting the size of the hole of the nozzle, the pressure applied to the liquid container, the number of times of pressing, and the like.

In a case where the liquid container is equipped with a spraying device, the spraying device has an end opening to which the nozzle is attached in the applicator of the present invention. Examples of such an applicator include the applicator shown in FIG. 4B. Also in this case, the applicator can apply a water-soluble sheet-like formulation held in the end portion of the nozzle to the skin, and supply a liquid to the back surface of the water-soluble sheet-like formulation by spraying the liquid from the spraying device. The amount of the liquid to be supplied can be appropriately set according to, for example, the size (area and thickness) of the water-soluble sheet-like formulation. The amount of the liquid can be set by adjusting the size of the hole of the nozzle, the discharge ability of the spraying device, the number of times of spraying, and the like.

In a case where the liquid container is equipped with a pump, the pump has an end opening to which the nozzle is attached in the applicator of the present invention. Examples of such an applicator include the applicator shown in FIG. 4A. Also in this case, the applicator can apply a water-soluble sheet-like formulation held in the end portion of the nozzle to the skin, and discharge a liquid to the back surface of the water-soluble sheet-like formulation by pushing the pump. The amount of the liquid to be discharged can be appropriately set according to, for example, the size (area and thickness) of the water-soluble sheet-like formulation. The amount of the liquid can be set by adjusting the size of the hole of the nozzle, the discharge ability of the pump, the number of times of discharge, and the like.

As described above, the present invention can provide an applicator of various aspects by using various liquid containers and attaching the nozzle with a foam ring edge of the present invention in a form such that the nozzle can be fitted into the opening of the liquid containers. Therefore, a nozzle and a liquid container can be freely combined according to the shape, the application purpose, the application frequency, the application site, and the like of the water-soluble sheet-like formulation. Furthermore, the nozzle with a foam ring edge of the present invention can be mass-produced at low cost, and therefore can be replaced and used for each subject and each application day. In the applicator after use, the nozzle with a foam ring edge is removed, the opening is sealed with a lid or the like, and the liquid remaining in the liquid container is refrigerated and stored. The liquid can be used again within the expiration date. Furthermore, the applicator of the present invention can also be used as a disposable device for one-time use in which the nozzle with a foam ring edge is attached to a liquid container filled with a liquid in an amount for one-time use.

Hereinafter, embodiments of the present invention will be described based on Examples. However, the present invention is not limited to the contents of Examples.

### EXAMPLES

### Example 1

A nozzle as shown in FIG. 1 was prepared. A silicone resin (one-component RTV rubber manufactured by Shin-Etsu Chemical Co., Ltd.) was used and subjected to injection molding to prepare a nozzle. The diameter of the flat surface of the nozzle end 1 was 12 mm, the level difference in the concentric uneven portions 3 was 0.5 mm, and the interval between the uneven portions was 1 mm. The upper view in FIG. 1 shows the section of the entire nozzle, and the lower view in FIG. 1 is the bottom view of the nozzle. The center of a nozzle body 10 is penetrated by a hole 2, and the hole 2 is visible at the center of the end portion. A liquid flows out from the hole 2, spreads into a groove 5, and spreads over the entire flat surface via the uneven portions 3. A urethane foam sheet (manufactured by O-BAN CO., LTD., custom-made product, expansion ratio: 5 times, thickness: 2.0 cm) was cut and punched into a circle having an outer diameter of 3.0 cm, and ring-like foam having a hole having a center inner diameter of 1.5 cm was prepared. The ring-like foam was fixed around the end of the nozzle, and set to be higher than the flat surface of the nozzle end by 1.0 mm.

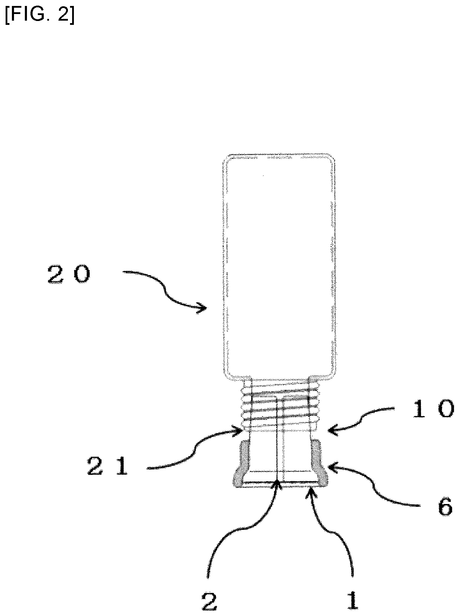

The resulting nozzle with a foam ring edge was attached to a polyethylene container (see FIG. 2), 0.1 g of LIMUVEIL HV (aqueous solution containing 1.2% of fucoidan, manufactured by TANGLEWOOD) was dissolved in 200 g of water, and the container was filled with the fucoidan-containing extract to prepare an applicator.

A microneedle patch was produced as follows. To 90 g of water, 5 g of hyaluronic acid (FCH-80, derived from culture, manufactured by Kikkoman Biochemifa Company) and 5 g of LIMUVEIL HV were added to prepare a uniform solution. The aqueous solution was cast on a mold, dried at room temperature for 24 hours, and taken out from the mold to obtain a microneedle array sheet. The sheet was cut to obtain a microneedle array having a diameter of 1 cm. One microneedle contained fucoidan at a content of 25 µg. The microneedle patch and the extract are formulations for the purpose of hair restoration.

Prior to use, the nozzle portion of the applicator was pressed against the back surface of the microneedle array to adhere the microneedle array to the nozzle portion and hold the microneedle array. The applicator was pressed against the forehead of a volunteer, and the container was squeezed to supply the fucoidan extract from the back surface of the microneedle array. This test was performed on 10 volunteers. When the applicator was pressed, the forehead was observed to confirm that no water flowed out of the nozzle surface on the 10 people.

### Comparative Example 1

A test was performed in the same manner as in Example 1 using a nozzle similar to that in Example 1 except that the nozzle had no ring edge. The container was squeezed to supply the fucoidan extract from the back surface of the microneedle array. This test was performed on 10 volunteers. When the applicator was pressed, the forehead was observed to confirm that water flowed out of the nozzle surface on 3 people among the 10 people.

### DESCRIPTION OF REFERENCE SYMBOLS

- 1: End portion of the nozzle
- 2: Hole
- 3: Uneven portion
- 4: Protrusion surrounding the periphery
- 5: Groove
- 6: Ring-like foam
- 10: Nozzle body
- 20: Liquid container
- 21: Opening
- 22: Pump
- 23: Spraying device

## Claims

1. A nozzle for hold and dissolution of a water-soluble sheet-like formulation, the nozzle comprising:
a nozzle having an end portion having a flat surface having a size such that a water-soluble sheet-like formulation can be held, the end portion having at least one hole, the flat surface being smooth excluding the at least one hole; and
ring-like foam disposed around a periphery of the end portion of the nozzle.

2. A nozzle for hold and dissolution of a water-soluble sheet-like formulation, the nozzle comprising:
a nozzle having an end portion having a flat surface having a size such that a water-soluble sheet-like formulation can be held, the end portion having at least one hole, the flat surface having an uneven portion excluding the at least one hole; and
ring-like foam disposed around a periphery of the end portion of the nozzle.

3. The nozzle for hold and dissolution of a water-soluble sheet-like formulation according to claim 2, wherein a level difference in the uneven portion is 0.1 to 3.0 mm.

4. The nozzle for hold and dissolution of a water-soluble sheet-like formulation according to claim 2 or 3, wherein the uneven portion forms a groove extending from the at least one hole toward the periphery of the end portion.

5. The nozzle for hold and dissolution of a water-soluble sheet-like formulation according to any one of claims 2 to 4, wherein the periphery of the end portion is surrounded by a protrusion higher than the uneven portion.

6. The nozzle for hold and dissolution of a water-soluble sheet-like formulation according to any one of claims 1 to 5, wherein the ring-like foam has an upper end higher than the periphery of the end portion of the nozzle by 0.1 to 10 mm in a state that the end portion of the nozzle is directed upward and the periphery of the end portion is in a horizontal direction.

7. The nozzle for hold and dissolution of a water-soluble sheet-like formulation according to any one of claims 1 to 6, wherein the nozzle includes a synthetic resin selected from the group consisting of polyethylene resins, polypropylene resins, soft polyolefin resins, polyurethane resins, polyvinyl chloride, soft polyvinyl chloride resins, and silicone resins.

8. The nozzle for hold and dissolution of a water-soluble sheet-like formulation according to any one of claims 1 to 7, wherein the nozzle includes a synthetic resin having self-viscosity.

9. The nozzle for hold and dissolution of a water-soluble sheet-like formulation according to any one of claims 1 to 8, wherein the nozzle includes a synthetic resin having self-viscosity, the synthetic resin selected from the group consisting of soft polyolefin resins, polyurethane resins, soft polyvinyl chloride resins, and silicone resins.

10. The nozzle for hold and dissolution of a water-soluble sheet-like formulation according to any one of claims 1 to 9, wherein the ring-like foam includes a material selected from the group consisting of polyurethanes, polyolefins, phenolic resins, polyvinyl chloride, and urea resins or includes a mixture of materials selected from the group consisting of polyurethanes, polyolefins, phenolic resins, polyvinyl chloride, and urea resins.

11. An applicator of a water-soluble sheet-like formulation, the applicator comprising:
a liquid container; and
the nozzle for hold and dissolution of a water-soluble sheet-like formulation according to any one of claims 1 to 10.

12. The applicator of a water-soluble sheet-like formulation according to claim 11, wherein the liquid container has an opening to which the nozzle is attached.

13. The applicator of a water-soluble sheet-like formulation according to claim 11 or 12, configured to apply a water-soluble sheet-like formulation held in the end portion of the nozzle to the skin, and supply a liquid to a back surface of the water-soluble sheet-like formulation by pressing the liquid container.

14. The applicator of a water-soluble sheet-like formulation according to claim 11, wherein the liquid container is equipped with a spraying device, and the spraying device has an end opening to which the nozzle is attached.

15. The applicator of a water-soluble sheet-like formulation according to claim 14, configured to apply a water-soluble sheet-like formulation held in the end portion of the nozzle to the skin, and supply a liquid to a back surface of the water-soluble sheet-like formulation by spraying the liquid from the spraying device.

16. The applicator of a water-soluble sheet-like formulation according to claim 11, wherein the liquid container is equipped with a pump, and the pump has an end opening to which the nozzle is attached.

17. The applicator of a water-soluble sheet-like formulation according to claim 16, configured to apply a water-soluble sheet-like formulation held in the end portion of the nozzle to the skin, and discharge a liquid to a back surface of the water-soluble sheet-like formulation by pushing the pump.
